# EUROPEAN PATENT APPLICATION

(11) **EP 4 749 404 A1**
(43) Date of publication of application: **27.05.2026**
(21) Application number: 24858601.8
(22) Date of filing: 28.08.2024
(51) Int. Cl.: G06F 3/01

(54) **INFORMATION PROCESSING METHOD AND DEVICE, AND SYSTEM**

(30) Priority: 03.09.2023 CN 202311133743
(71) Applicant: HUAWEI TECHNOLOGIES CO., LTD., Shenzhen, Guangdong 518129 (CN)
(72) Inventor: HUANG, Heming, Shenzhen, Guangdong 518129 (CN); CHEN, Yun, Shenzhen, Guangdong 518129 (CN); TIAN, Kun, Shenzhen, Guangdong 518129 (CN); ZHANG, Jun, Shenzhen, Guangdong 518129 (CN); WANG, Tieying, Shenzhen, Guangdong 518129 (CN); LIAO, Jianxing, Shenzhen, Guangdong 518129 (CN)
(74) Representative: Epping - Hermann - Fischer
(86) International application number: PCT/CN2024/115018
(87) International publication number: WO 2025/045067

(57) **Abstract**

This application provides an information processing method, apparatus, and system, and belongs to the computer field. The method is applied to a brain-computer system, the brain-computer system includes a control module (102), and the method includes: The control module (102) receives a first signal sent by a signal collection module (101), where the first signal is obtained by the signal collection module (101) by converting a first neural signal of a brain based on a first configuration parameter; and when the first signal is in an abnormal state, the control module (102) sends first information, where the first information indicates the signal collection module to adjust the first configuration parameter to a second configuration parameter. This application ensures that a feedback module (103) can be controlled.

## Description

This application claims priority to Chinese Patent Application No. 202311133743.1, filed on September 3, 2023 and entitled "INFORMATION PROCESSING METHOD, APPARATUS, AND SYSTEM", which is incorporated herein by reference in its entirety.

### TECHNICAL FIELD

This application relates to the computer field, and in particular, to an information processing method, apparatus, and system.

### BACKGROUND

A brain-computer interface creates a connection between a brain and an external device to implement information exchange between the brain and a feedback device. The brain-computer interface may collect a neural signal generated by the brain, and may obtain a brain instruction or a brain status based on the neural signal. The brain instruction may reflect an idea of the brain, and the brain status may reflect a physiological condition of the brain. In this way, the feedback device is controlled based on the brain instruction or the brain status to perform a corresponding operation. In related technologies, the brain-computer interface includes a signal collection module and a control device, and the control device communicates with the signal collection module and the feedback device. The signal collection module is configured to: collect the neural signal in the brain, convert the neural signal into a digital signal, and send the digital signal to the control device. The control device obtains the brain instruction or the brain status based on the digital signal, and controls, based on the brain instruction, the feedback device to implement the idea of the brain, or controls, based on the brain status, the feedback device to intervene in the physiological condition of the brain.

In the related technologies, the digital signal obtained by the signal collection module through conversion may be abnormal. As a result, the control device cannot obtain the brain instruction or the brain status based on the digital signal, and consequently cannot control the feedback device.

### SUMMARY

This application provides an information processing method, apparatus, and system, to ensure that a feedback module can be controlled. The technical solutions are as follows.

According to a first aspect, this application provides an information processing method, where the method is applied to a brain-computer system, and the brain-computer system includes a control module. In the method, the control module receives a first signal sent by a signal collection module, where the first signal is obtained by the signal collection module by converting a first neural signal of a brain based on a first configuration parameter. When the first signal is in an abnormal state, the control module sends first information, where the first information indicates the signal collection module to adjust the first configuration parameter to a second configuration parameter. Because the control module sends the first information when the first signal is in the abnormal state, where the first information indicates the signal collection module to adjust the first configuration parameter to the second configuration parameter, a proper configuration parameter can be obtained through adjustment. In this way, the signal collection module obtains the first signal in a normal state through conversion based on the proper configuration parameter, to ensure that a brain instruction or a brain status can be obtained based on the first signal, and ensure that a feedback module can be controlled.

In a possible implementation, the abnormal state includes at least one of signal saturation, low signal amplitude, and an oscillation state.

In another possible implementation, the control module includes a threshold determining unit, a mode determining unit, and a storage unit. The threshold determining unit is configured to: compare an amplitude threshold with an amplitude of the first signal to obtain a comparison result, where the amplitude threshold includes a first amplitude threshold and a second amplitude threshold, and the first amplitude threshold is greater than the second amplitude threshold; store the comparison result in the storage unit; and when the comparison result is that the amplitude of the first signal exceeds the first amplitude threshold, determine a status of the first signal, where the status of the first signal includes signal saturation, the storage unit stores a plurality of comparison results, and the plurality of comparison results are comparison results obtained by the threshold determining unit at different time. The mode determining unit is configured to: when determining, based on the storage unit, that the signal saturation periodically occurs, determine that the status of the first signal further includes an oscillation state; or when determining, based on the storage unit, that a plurality of consecutive comparison results are all that the amplitude of the first signal is less than the second amplitude threshold, determine that the status of the first signal includes low signal amplitude. In this way, the status of the first signal can be obtained, so that whether the first signal is in the abnormal state can be determined.

In another possible implementation, the threshold determining unit is implemented by using software or a hardware circuit, and the mode determining unit is implemented by using software or a hardware circuit.

In another possible implementation, the signal collection module includes a main control unit, a calculation unit, and a signal conversion unit, and the first signal is obtained by the signal conversion unit by converting the first neural signal based on the first configuration parameter. The first information indicates the main control unit to send an adjustment manner to the calculation unit based on the status. The adjustment manner indicates the calculation unit to adjust the first configuration parameter to the second configuration parameter. In this way, the proper configuration parameter can be obtained through adjustment, so that the signal collection module obtains the first signal in the normal state through conversion based on the proper configuration parameter, to ensure that the brain instruction or the brain status can be obtained based on the first signal, and ensure that the feedback module can be controlled.

In another possible implementation, when the first signal is in a normal state, the control module obtains the brain instruction based on the first signal, where the brain instruction indicates an action that needs to be performed for the brain. The control module controls, based on the brain instruction, the feedback module to implement the action. In this way, it is ensured that the feedback module can be controlled to perform the action.

In another possible implementation, when the first signal is in a normal state, the control module obtains a first brain state based on the first signal, where the first brain state is used to reflect a physiological condition that occurs in the brain. The control module controls, based on the first brain state, the feedback module to intervene in the physiological condition. In this way, it is ensured that the feedback module can be controlled to intervene in the physiological condition.

In another possible implementation, the control module receives a second signal, where the second signal is obtained by the signal collection module by converting a second neural signal generated by the brain, and the second neural signal is collected after the first neural signal is collected. The control module obtains a second brain state based on the second signal. When determining, based on the second brain state, that the physiological condition still occurs in the brain, the control module adjusts an intervention parameter in the feedback module, where the intervention parameter is a parameter used by the feedback module to intervene in the physiological condition. In this way, when the intervention parameter in the feedback module is inappropriate, the intervention parameter can be automatically adjusted.

In another possible implementation, the control module and the signal collection module are integrated into one device, so that a structure of the brain-computer system can be simplified.

In another possible implementation, the control module is implemented by using software or a hardware circuit, and the signal collection module is implemented by using software or a hardware circuit.

According to a second aspect, this application provides an information processing method, where the method is applied to a brain-computer system, and the brain-computer system includes a control module. In the method, the control module sends a control command, where the control command indicates a feedback module to intervene in a physiological condition that occurs in a brain. The control module receives feedback information, where the feedback information indicates a situation in which the feedback module intervenes in the physiological condition. The control module determines, based on the feedback information, an abnormality that occurs in the feedback module. The feedback module sends the feedback information to the control module, where the feedback information indicates a situation in which the feedback module intervenes in the physiological condition. The control module determines, based on the feedback information, the abnormality that occurs in the feedback module, so that the abnormality can be positioned.

In a possible implementation, the abnormality means that the feedback module does not intervene in the physiological condition, or the abnormality means that the feedback module incorrectly intervenes in the physiological condition.

In another possible implementation, the abnormality means that the feedback module incorrectly intervenes in the physiological condition, the feedback information indicates whether a first intervention parameter is the same as a second intervention parameter, the first intervention parameter is a parameter expected to be used by the feedback module to intervene in the physiological condition, and the second intervention parameter is a parameter actually used by the feedback module to intervene in the physiological condition. When determining, based on the feedback information, that the first intervention parameter is different from the second intervention parameter, the control module determines that the feedback module incorrectly intervenes in the physiological condition, so that an abnormality of the incorrect intervention can be positioned.

In another possible implementation, the feedback information includes the second intervention parameter; or the feedback module includes the first intervention parameter, and the feedback information includes a comparison result of comparing the first intervention parameter with the second intervention parameter by the feedback module.

In another possible implementation, the feedback module includes a control driving unit and a monitoring unit. The control driving unit is configured to intervene in the physiological condition. The monitoring unit is configured to: when detecting that the control driving unit intervenes in the physiological condition, obtain the second intervention parameter, and send the feedback information, where the feedback information includes the second intervention parameter, or a comparison result of comparing the first intervention parameter with the second intervention parameter.

According to a third aspect, this application provides an information processing method, where the method is applied to a brain-computer system, and the brain-computer system includes a signal collection module. In the method, the signal collection module receives second information, where the second information indicates a feedback module to intervene in a physiological condition that occurs in a brain, and the physiological condition is obtained based on a neural signal that is of the brain and that is collected by the signal collection module. The signal collection module performs a first operation based on the second information, where the first operation is used to reduce impact of an intervention operation performed by the feedback module on collection of the neural signal. The feedback module sends the second information to the signal collection module, and performs the first operation based on the second information. The first operation includes one or more of the following: disabling a function of collecting the neural signal, or decreasing an amplification multiple for the neural signal. In this way, a control module can stop controlling the feedback module to continue to intervene in the physiological condition of the brain, to avoid controlling the feedback module to perform incorrect intervention.

In another possible implementation, the first operation includes one or more of the following: disabling a function of collecting the neural signal, or decreasing an amplification multiple for the neural signal.

According to a fourth aspect, this application provides an information processing apparatus, configured to perform the method in any one of the first aspect or the possible implementations of the first aspect. Specifically, the apparatus includes a unit configured to perform the method in any one of the first aspect or the possible implementations of the first aspect.

According to a fifth aspect, this application provides an information processing apparatus, configured to perform the method in any one of the second aspect or the possible implementations of the second aspect. Specifically, the apparatus includes a unit configured to perform the method in any one of the second aspect or the possible implementations of the second aspect.

According to a sixth aspect, this application provides an information processing apparatus, configured to perform the method in any one of the third aspect or the possible implementations of the third aspect. Specifically, the apparatus includes a unit configured to perform the method in any one of the third aspect or the possible implementations of the third aspect.

According to a seventh aspect, this application provides a computer program product. The computer program product includes a computer program stored in a computer-readable storage medium, and the computer program is loaded by a processor to implement instructions of the method in any one of the first aspect, the second aspect, the third aspect, the possible implementations of the first aspect, the possible implementations of the second aspect, or the possible implementations of the third aspect.

According to an eighth aspect, this application provides a computer-readable storage medium, configured to store a computer program. The computer program is loaded by a processor to execute instructions of the method in any one of the first aspect, the second aspect, the third aspect, the possible implementations of the first aspect, the possible implementations of the second aspect, or the possible implementations of the third aspect.

According to a ninth aspect, this application provides a chip, including a memory and a processor. The memory is configured to store computer instructions, and the processor is configured to invoke the computer instructions from the memory and run the computer instructions, to execute instructions of the method in any one of the first aspect, the second aspect, the third aspect, the possible implementations of the first aspect, the possible implementations of the second aspect, or the possible implementations of the third aspect.

According to a tenth aspect, this application provides an information processing system. The system includes the apparatus according to the fourth aspect, the apparatus according to the fifth aspect, and the apparatus according to the sixth aspect.

### BRIEF DESCRIPTION OF DRAWINGS

FIG. 1 is a diagram of a structure of a brain-computer system according to an embodiment of this application;
FIG. 2 is a diagram of a structure of another brain-computer system according to an embodiment of this application;
FIG. 3 is a diagram of a structure of another brain-computer system according to an embodiment of this application;
FIG. 4 is a diagram of a structure of another brain-computer system according to an embodiment of this application;
FIG. 5 is a flowchart of an information processing method according to an embodiment of this application;
FIG. 6 is a flowchart of another information processing method according to an embodiment of this application;
FIG. 7 is a flowchart of another information processing method according to an embodiment of this application;
FIG. 8 is a diagram of a structure of an information processing apparatus according to an embodiment of this application;
FIG. 9 is a diagram of a structure of another information processing apparatus according to an embodiment of this application;
FIG. 10 is a diagram of a structure of another information processing apparatus according to an embodiment of this application;
FIG. 11 is a diagram of a structure of another information processing apparatus according to an embodiment of this application; and
FIG. 12 is a diagram of a structure of another information processing apparatus according to an embodiment of this application.

### DESCRIPTION OF EMBODIMENTS

The following further describes in detail the implementations of this application with reference to the accompanying drawings.

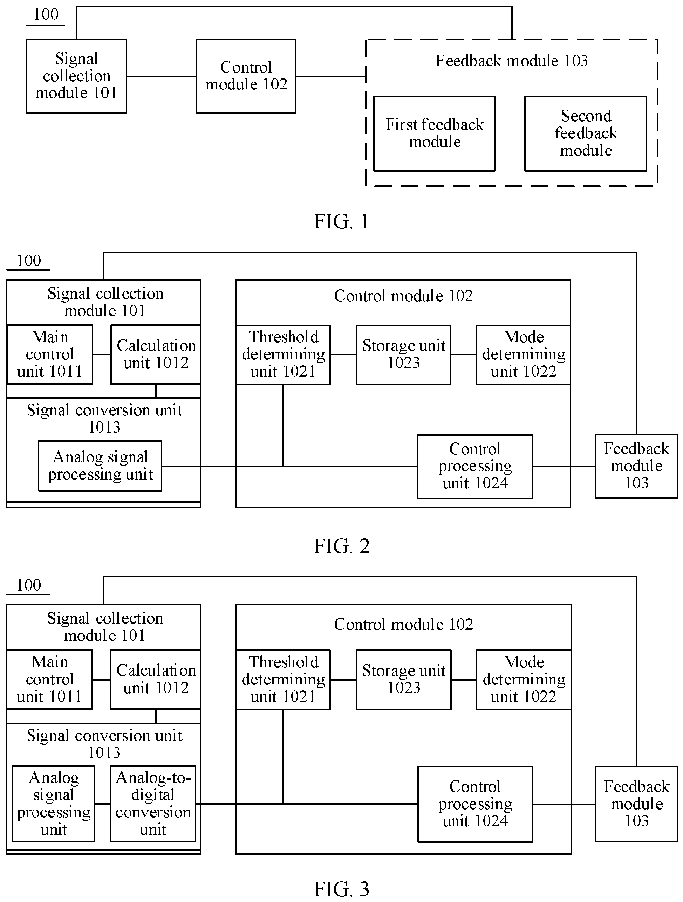

Refer to FIG. 1. An embodiment of this application provides a brain-computer system 100, and the brain-computer system 100 includes a signal collection module 101, a control module 102, at least one feedback module 103, and the like. The control module 102 may separately communicate with the signal collection module 101 and the at least one feedback module 103, and the signal collection module 101 may further communicate with the at least one feedback module 103 or some of the at least one feedback module 103.

In some embodiments, the modules in this embodiment of this application may be implemented by using software or hardware circuits. For example, the signal collection module 101 may be implemented by using software or a hardware circuit. For example, the signal collection module 101 may be a signal collection circuit. For another example, the control module 102 may be implemented by using software or a hardware circuit. For example, the control module 102 may be a control circuit. For still another example, the feedback module 103 may be implemented by using software or a hardware circuit. For example, the feedback module 103 may be a feedback circuit. In this embodiment of this application, meanings of other modules are the same as meanings of the modules herein, and are not described one by one again.

In some embodiments, the at least one feedback module 103 may include at least one first feedback module and/or at least one second feedback module. The first feedback module is configured to implement an action that needs to be performed for a brain, and the second feedback module is configured to intervene in a physiological condition that occurs in the brain.

For example, when there is a need of doing housework, the first feedback module may be a device like a robot configured to do housework; when there is a need of watching TV, the first feedback module may be a television; or when there is a need of listening to music, the first feedback module may be a music player. The first feedback module may alternatively be another device, and details are not listed one by one herein.

The second feedback module may be a treatment device configured to treat a brain disease. For example, the second feedback module may be a nerve stimulator configured to treat epilepsy. Alternatively, the second feedback module may be an assistance device configured to assist in sleep. For example, the second feedback module is a sleep aid device. The second feedback module may alternatively be another device, and details are not listed one by one herein.

The signal collection module 101 may collect a first neural signal of the brain, convert the first neural signal into a first signal based on a first configuration parameter, and send the first signal to the control module 102.

The control module 102 may receive the first signal, and obtain a brain instruction or a brain status based on the first signal. The brain instruction is used to reflect the action that needs to be performed for the brain, and the brain status is used to reflect a physiological condition that occurs in the brain. The control module 102 controls, based on the brain instruction, the first feedback module to implement the action, or controls, based on the brain status, the second feedback module to intervene in the physiological condition that occurs in the brain.

In some embodiments, the control module 102 includes a decoding algorithm, and the control module 102 processes the first signal by using the decoding algorithm, to obtain the brain instruction or the brain status.

In some embodiments, the first signal may be an electrical signal. The control module 102 receives the first signal, converts the first signal into a digital signal, and processes the digital signal by using the decoding algorithm, to obtain the brain instruction or the brain status.

In some embodiments, the first signal may be a digital signal. The control module 102 receives the first signal, and processes the first signal by using the decoding algorithm, to obtain the brain instruction or the brain status.

In some embodiments, the first signal may be in an abnormal state. Consequently, the control module 102 may fail to obtain the brain instruction or the brain status based on the first signal. The abnormal state of the first signal may be caused by an improper first configuration parameter in the signal collection module 101.

When the first signal is in the abnormal state, the control module 102 may further send first information to the signal collection module 101. The signal collection module 101 receives the first information, and adjusts the first configuration parameter to a second configuration parameter based on the first information, so that the second configuration parameter becomes proper.

In some embodiments, refer to FIG. 2. The signal collection module 101 includes a main control unit 1011, a calculation unit 1012, and a signal conversion unit 1013.

The signal conversion unit 1013 is configured to: collect the first neural signal of the brain, convert the collected first neural signal into the first signal based on the first configuration parameter, and send the first signal to the control module 102.

The main control unit 1011 may receive the first information sent by the control module 102, and send an adjustment manner to the calculation unit 1012 based on the first information. The calculation unit 1012 adjusts the first configuration parameter in the signal conversion unit 1013 to the second configuration parameter based on the adjustment manner. The adjustment manner may be decreasing the first configuration parameter or increasing the first configuration parameter. In some embodiments, the units in this embodiment of this application may be implemented by using software or hardware circuits. For example, the main control unit 1011 may be implemented by using software or a hardware circuit. For example, the main control unit 1011 may be a main control circuit. For another example, the calculation unit 1012 may be implemented by using software or a hardware circuit. For example, the calculation unit 1012 may be a calculation circuit. For still another example, the signal conversion unit 1013 may be implemented by using software or a hardware circuit. For example, the signal conversion unit 1013 may be a signal conversion circuit. In this embodiment of this application, meanings of other units are the same as meanings of the units herein, and are not described one by one again.

In some embodiments, refer to FIG. 2. The signal conversion unit 1013 includes an analog signal processing unit and the like. The analog signal processing unit may convert the first neural signal into an electrical signal, perform processing such as amplification and/or filtering on the electrical signal based on the first configuration parameter, and send the first signal to the control module 102, where the first signal is a processed electrical signal.

In some embodiments, refer to FIG. 3. The signal conversion unit 1013 includes an analog signal processing unit, an analog-to-digital conversion unit, and the like. The analog signal processing unit may convert the first neural signal into an electrical signal, and send the electrical signal to the analog-to-digital conversion unit. Optionally, the analog signal processing unit may perform processing such as amplification and/or filtering on the electrical signal based on a part or all of parameters among the first configuration parameter, and send a processed electrical signal to the analog-to-digital conversion unit.

The analog-to-digital conversion unit receives the electrical signal sent by the analog signal processing unit, converts the electrical signal into the first signal, where the first signal is a digital signal, and sends the first signal to the control module 102. Optionally, the analog-to-digital conversion unit may perform processing such as amplification and/or filtering on the first signal based on a part or all of the parameters among the first configuration parameter, and send a processed first signal to the control module 102.

Optionally, the analog-to-digital conversion unit may be a circuit like an analog-to-digital converter (analog-to-Digital Converter, ADC).

In some embodiments, refer to FIG. 2 or FIG. 3. The control module 102 includes a threshold determining unit 1021, a mode determining unit 1022, and a storage unit 1023.

The threshold determining unit 1021 may receive the first signal, and compare an amplitude threshold with an amplitude of the first signal to obtain a comparison result, where the amplitude threshold includes a first amplitude threshold and a second amplitude threshold, and the first amplitude threshold is greater than the second amplitude threshold; store the comparison result in the storage unit 1023; and when the comparison result is that the amplitude of the first signal exceeds the first amplitude threshold, determine a status of the first signal, where the status of the first signal includes signal saturation, the storage unit 1023 stores a plurality of comparison results, and the plurality of comparison results are comparison results obtained by the threshold determining unit 1021 at different time.

The mode determining unit 1022 is configured to: when determining, based on the storage unit 1023, that the signal saturation periodically occurs, determine that the status of the first signal further includes an oscillation state; or when determining, based on the storage unit 1023, that a plurality of consecutive comparison results are all that the amplitude of the first signal is less than the second amplitude threshold, determine that the status of the first signal includes low signal amplitude.

In some embodiments, refer to FIG. 2 or FIG. 3. The control module 102 further includes a control processing unit 1024.

The control processing unit 1024 may receive the first signal, and obtain the brain instruction or the brain status based on the first signal. The brain instruction is used to reflect the action that needs to be performed for the brain, and the brain status is used to reflect the physiological condition that occurs in the brain. The control processing unit 1024 controls, based on the brain instruction, the first feedback module to implement the action, or controls, based on the brain status, the second feedback module to intervene in the physiological condition that occurs in the brain. In some embodiments, the first signal is a digital signal, and the control processing unit 1024 obtains the brain instruction or the brain status based on the digital signal. Alternatively, the first signal is an electrical signal, and the control processing unit 1024 converts the first signal into a digital signal, and obtains the brain instruction or the brain status based on the digital signal.

In some embodiments, the first signal may be highly noisy, and the first signal is a digital signal. The control processing unit 1024 may increase a signal-to-noise ratio of the first signal, and process, by using the decoding algorithm, the first signal with an increased signal-to-noise ratio, to obtain the brain instruction or the brain status. Alternatively, the first signal is an electrical signal. The control processing unit 1024 may convert the first signal into a digital signal, increase a signal-to-noise ratio of the digital signal, and process, by using the decoding algorithm, the digital signal with an increased signal-to-noise ratio, to obtain the brain instruction or the brain status.

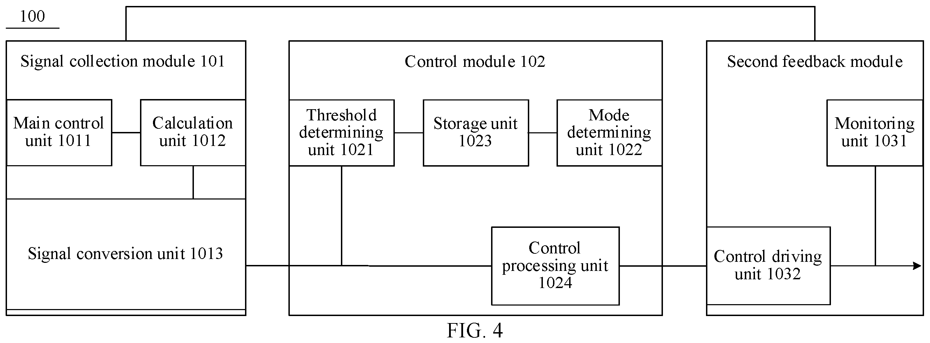

In some embodiments, refer to FIG. 4. The second feedback module may include a monitoring unit 1031 and a control driving unit 1032.

When obtaining the brain status based on the first signal, the control module 102 sends a control command to the second feedback module.

The control driving unit 1032 receives the control command, and intervenes in, based on the control command, the physiological condition that occurs in the brain.

The monitoring unit 1031 may monitor a situation in which the second feedback module intervenes in the physiological condition, and send feedback information to the control module 102.

The control module 102 determines, based on the feedback information, an abnormality that occurs in the second feedback module.

In some embodiments, the monitoring unit 1031 may further send second information to the signal collection module 101, where the second information indicates the second feedback module to intervene in the physiological condition that occurs in the brain.

The signal collection module 101 may perform a first operation based on the second information, where the first operation is used to reduce impact of an intervention operation performed by the second feedback module on collection of a neural signal.

In some embodiments, the main control unit 1011 in the signal collection module 101 may receive the second information, and perform the first operation based on the second information.

In some embodiments, the signal collection module 101 and the control module 102 may be integrated into one device, or the signal collection module 101 and the control module 102 are not integrated into one device.

In some embodiments, the signal collection module 101 may be a chip, a helmet, a probe, or the like.

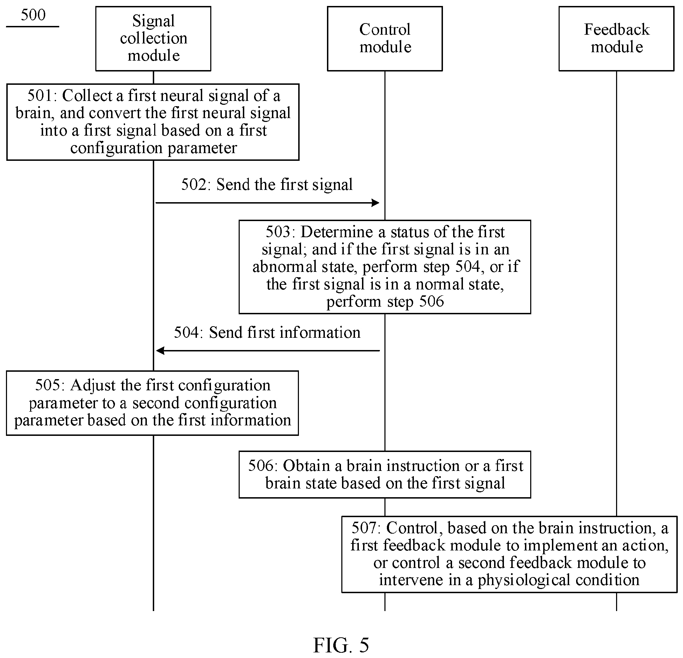

Refer to FIG. 5. An embodiment of this application provides an information processing method 500. The method 500 is applied to the brain-computer system 100 shown in FIG. 1, FIG. 2, FIG. 3, or FIG. 4. When a configuration parameter in a signal collection module is improper, the method 500 may be used to adjust the configuration parameter in the signal collection module to a proper configuration parameter. The method 500 includes the following procedure.

Step 501: The signal collection module collects a first neural signal of a brain, and converts the first neural signal into a first signal based on a first configuration parameter.

In some embodiments, the first configuration parameter includes at least one amplification multiple. Optionally, the first configuration parameter includes one amplification multiple, or the first configuration parameter includes a first amplification multiple and a second amplification multiple.

The first signal may be an electrical signal or a digital signal. In step 501, the signal collection module may convert the first neural signal into the first signal in the following two manners. Certainly, there may be other manners of converting the first neural signal into the first signal, and the manners are not listed one by one herein.

Manner 1: The first signal is a digital signal, the first configuration parameter includes the amplification multiple, and the signal collection module converts the first neural signal into an electrical signal, and converts the electrical signal into a digital signal. The digital signal is amplified based on the amplification multiple, and the first signal is an amplified digital signal. Alternatively, the first configuration parameter may further include a filtering parameter (for example, filtering duration and/or a filtering frequency), the amplified digital signal is filtered based on the filtering parameter, and the first signal is a filtered digital signal.

In some embodiments, the signal collection module may be the signal collection module in the brain-computer system 100 shown in FIG. 3. A signal conversion unit in the signal collection module includes an analog signal processing unit, an analog-to-digital conversion unit, and the like. The following lists several examples in which the signal collection module converts the first neural signal into the first signal. Certainly, there are other examples in which the first neural signal is converted into the first signal, and the examples are not listed one by one herein.

Example 1: The first configuration parameter includes one amplification multiple. The analog signal processing unit may convert the first neural signal into an electrical signal, amplify the electrical signal based on the amplification multiple, and send an amplified electrical signal to the analog-to-digital conversion unit. The analog-to-digital conversion unit receives the amplified electrical signal, and converts the amplified electrical signal into a digital signal, and the first signal is the digital signal. Optionally, the first configuration parameter may further include the filtering parameter, the digital signal is filtered based on the filtering parameter, and the first signal is a filtered digital signal.

Example 2: The first configuration parameter includes one amplification multiple. The analog signal processing unit may convert the first neural signal into an electrical signal, and send the electrical signal to the analog-to-digital conversion unit. The analog-to-digital conversion unit receives the electrical signal, converts the electrical signal into a digital signal, and amplifies the digital signal based on the amplification multiple to obtain the first signal. The first signal is an amplified digital signal. Optionally, the first configuration parameter may further include the filtering parameter, the amplified digital signal is filtered based on the filtering parameter, and the first signal is a filtered digital signal.

Example 3: The first configuration parameter includes the first amplification multiple and the second amplification multiple. The analog signal processing unit may convert the first neural signal into an electrical signal, amplify the electrical signal based on the first amplification multiple, and send an amplified electrical signal to the analog-to-digital conversion unit. The analog-to-digital conversion unit receives the electrical signal, converts the electrical signal into a digital signal, and amplifies the digital signal based on the second amplification multiple to obtain the first signal. The first signal is an amplified digital signal. Optionally, the first configuration parameter may further include the filtering parameter, the amplified digital signal is filtered based on the filtering parameter, and the first signal is a filtered digital signal.

Manner 2: The first signal is an electrical signal, the first configuration parameter includes the amplification multiple, and the signal collection module converts the first neural signal into an electrical signal. The electrical signal is amplified based on the amplification multiple, and the first signal is an amplified electrical signal. Alternatively, the first configuration parameter may further include a filtering parameter, the amplified electrical signal is filtered based on the filtering parameter, and the first signal is a filtered electrical signal.

In some embodiments, in Manner 2, the signal collection module may be the signal collection module 101 in the brain-computer system 100 shown in FIG. 2. An analog signal processing unit included in the signal collection module 101 converts the first neural signal into an electrical signal, and amplifies the electrical signal based on the amplification multiple.

Step 502: The signal collection module sends the first signal to a control module.

The signal collection module and the control module are not integrated into one device, the signal collection module and the control module are connected in a wired manner, and the signal collection module may send the first signal to the control module. Alternatively, a wireless communication connection is established between the signal collection module and the control module, and the signal collection module may send the first signal to the control module through the wireless communication connection.

The signal collection module and the control module are integrated into one device, the signal collection module and the control module are connected through an internal connection like a bus or a board, and the signal collection module may send the first signal to the control module through the internal connection.

Step 503: The control module receives the first signal, and determines a status of the first signal; and if the first signal is in an abnormal state, performs step 504, or if the first signal is in a normal state, performs step 506.

The abnormal state includes at least one of signal saturation, low signal amplitude, and an oscillation state.

The signal saturation means that an amplitude of the first signal exceeds a first amplitude threshold. Low signal amplitude means that the amplitude of the first signal is less than a second amplitude threshold, and the first amplitude threshold is greater than the second amplitude threshold. The oscillation state means that the signal saturation periodically occurs.

In step 503, the control module compares an amplitude threshold with the amplitude of the first signal to obtain a comparison result, and stores the comparison result. The control module may have stored a comparison result obtained before current time.

The amplitude threshold includes the first amplitude threshold and the second amplitude threshold, and the control module compares the amplitude of the first signal with the first amplitude threshold to obtain a comparison result. When the comparison result is that the amplitude of the first signal exceeds the first amplitude threshold, it is determined that the status of the first signal includes the signal saturation. When it is determined, based on a plurality of stored comparison results, that a plurality of consecutive comparison results are all that the amplitude of the first signal is less than the second amplitude threshold, it is determined that the status of the first signal includes low signal amplitude. When the comparison result is that the amplitude of the first signal exceeds the second amplitude threshold but does not exceed the first amplitude threshold, it is determined that the first signal is in a normal state.

The control module further stores the status of the first signal. Statuses of the signal obtained by the control module at different time are stored, that is, the control module stores a plurality of statuses. If the signal saturation periodically occurs in the plurality of statuses, it is determined that the status of the first signal further includes the oscillation state.

Refer to FIG. 2, FIG. 3, or FIG. 4. The control module includes a threshold determining unit, a mode determining unit, and a storage unit.

The threshold determining unit is configured to: compare the amplitude threshold with the amplitude of the first signal to obtain the comparison result, where the amplitude threshold includes the first amplitude threshold and the second amplitude threshold; store the comparison result in the storage unit; and when the comparison result is that the amplitude of the first signal exceeds the first amplitude threshold, determine the status of the first signal, where the status of the first signal includes the signal saturation, the storage unit stores a plurality of comparison results, and the plurality of comparison results are comparison results obtained by the threshold determining unit at different time.

The mode determining unit is configured to: when determining, based on the storage unit, that the signal saturation periodically occurs, determine that the status of the first signal further includes the oscillation state; or when determining, based on the storage unit, that a plurality of consecutive comparison results are all that the amplitude of the first signal is less than the second amplitude threshold, determine that the status of the first signal includes low signal amplitude.

The status of the first signal includes the signal saturation and/or the oscillation state. It indicates that power of the first signal is excessively high, and consequently, the control module cannot parse the first signal. In this case, an amplification multiple for the first signal needs to be decreased. Alternatively, the status of the first signal includes low signal amplitude. It indicates that power of the first signal is excessively low, and consequently, the control module cannot parse the first signal. In this case, an amplification multiple for the first signal needs to be increased.

Step 504: The control module sends first information to the signal collection module.

In some embodiments, the first information includes the status of the first signal, or the control module obtains an adjustment manner based on the status of the first signal. The first information includes the adjustment manner, and the adjustment manner is decreasing the first configuration parameter or increasing the first configuration parameter.

In some embodiments, when the status of the first signal includes the signal saturation and/or the oscillation state, the adjustment manner obtained by the control module is decreasing the first configuration parameter; or when the status of the first signal includes low signal amplitude, the obtained adjustment manner is increasing the first configuration parameter.

Step 505: The signal collection module receives the first information, adjusts the first configuration parameter to a second configuration parameter based on the first information, and returns to perform step 501.

In step 505, the signal collection module receives the first information, obtains the adjustment manner based on the first information, and adjusts the first configuration parameter to the second configuration parameter based on the adjustment manner.

In some embodiments, the first information includes the status of the first signal, and the signal collection module obtains the adjustment manner based on the status of the first signal, and adjusts the first configuration parameter to the second configuration parameter based on the adjustment manner. Alternatively, the first information includes the adjustment manner, and the first configuration parameter is adjusted to the second configuration parameter based on the adjustment manner.

Optionally, the adjustment manner is decreasing the first configuration parameter. The signal collection module decreases the amplification multiple included in the first configuration parameter to obtain the second configuration parameter. Alternatively, the adjustment manner is increasing the first configuration parameter. The signal collection module increases the amplification multiple included in the first configuration parameter to obtain the second configuration parameter.

The following lists two manners of adjusting the first configuration parameter to the second configuration parameter. Certainly, there may be other manners of adjusting the first configuration parameter to the second configuration parameter, and the manners are not listed one by one herein. Manner 1: The adjustment manner is decreasing the first configuration parameter. The signal collection module decreases the amplification multiple included in the first configuration parameter by a first value to obtain the second configuration parameter. Alternatively, the adjustment manner is increasing the first configuration parameter. The signal collection module increases the amplification multiple included in the first configuration parameter by a second value to obtain the second configuration parameter. Optionally, both the first value and the second value are preset values.

In some embodiments, the signal collection module may be the signal collection module in the brain-computer system 100 shown in FIG. 2 or FIG. 3. The signal conversion unit includes a main control unit, a calculation unit, and a signal conversion unit, and the signal conversion unit includes the first configuration parameter.

In Manner 1, the main control unit receives the first information, obtains the adjustment manner based on the first information, and sends the adjustment manner to the calculation unit. Optionally, the first information includes the status of the first signal, and the main control unit obtains the adjustment manner based on the status of the first signal; or the first information includes the adjustment manner, and the main control unit obtains the adjustment manner in the first information.

The calculation unit receives the adjustment manner, obtains the first configuration parameter included in the signal conversion unit. When the adjustment manner is decreasing the first configuration parameter, the calculation unit decreases the amplification multiple included in the first configuration parameter by the first value to obtain the second configuration parameter, and updates the first configuration parameter in the signal conversion unit to the second configuration parameter. Alternatively, when the adjustment manner is increasing the first configuration parameter, the calculation unit increases the amplification multiple included in the first configuration parameter by the second value to obtain the second configuration parameter, and updates the first configuration parameter in the signal conversion unit to the second configuration parameter.

Manner 2: The signal collection module includes a plurality of configuration parameters, the plurality of configuration parameters include the first configuration parameter, and the adjustment manner is decreasing the first configuration parameter. The signal collection module selects, from the plurality of configuration parameters, a configuration parameter less than the first configuration parameter as the second configuration parameter. Alternatively, the adjustment manner is increasing the first configuration parameter. The signal collection module selects, from the plurality of configuration parameters, a configuration parameter greater than the first configuration parameter as the second configuration parameter.

In some embodiments, the signal collection module may be the signal collection module in the brain-computer system 100 shown in FIG. 2 or FIG. 3. The signal conversion unit includes a main control unit, a calculation unit, and a signal conversion unit, and the signal conversion unit includes the first configuration parameter.

In Manner 2, the main control unit receives the first information, obtains the adjustment manner based on the first information, and sends the adjustment manner to the calculation unit.

The calculation unit receives the adjustment manner, and obtains the first configuration parameter included in the signal conversion unit, where the calculation unit includes a plurality of configuration parameters. When the adjustment manner is decreasing the first configuration parameter, the calculation unit selects, from the plurality of configuration parameters, a configuration parameter less than the first configuration parameter as the second configuration parameter, and updates the first configuration parameter in the signal conversion unit to the second configuration parameter. Alternatively, when the adjustment manner is increasing the first configuration parameter, the calculation unit selects, from the plurality of configuration parameters, a configuration parameter greater than the first configuration parameter as the second configuration parameter, and updates the first configuration parameter in the signal conversion unit to the second configuration parameter.

After adjusting the first configuration parameter to the second configuration parameter, the signal collection module may continue to collect a neural signal of the brain, and convert the neural signal based on the second configuration parameter to obtain the first signal, that is, may continue to perform step 501. If the first signal is still in the abnormal state, the second configuration parameter continues to be adjusted according to step 501 to step 505, until the second configuration parameter is adjusted to a proper configuration parameter. In this way, the signal collection module can obtain the first signal in the normal state, and send the first signal in the normal state to the control module, so that the control module can obtain a brain instruction or a brain status based on the first signal in the normal state, to successfully control a feedback module.

Step 506: The control module obtains the brain instruction or a first brain state based on the first signal, where the brain instruction indicates an action that needs to be performed for the brain, and the first brain state is used to reflect a physiological condition that occurs in the brain.

In step 506, the control module may increase a signal-to-noise ratio of the first signal, and process, by using a decoding algorithm, the first signal with an increased signal-to-noise ratio, to obtain the brain instruction or the first brain state. Optionally, the control module may extract at least one information feature from the first signal by using the decoding algorithm, and obtain the brain instruction or the first brain state based on the at least one information feature.

Refer to FIG. 2, FIG. 3, or FIG. 4. The control module includes a control processing unit. The control processing unit may increase the signal-to-noise ratio of the first signal, and process, by using the decoding algorithm, the first signal with the increased signal-to-noise ratio, to obtain the brain instruction or the brain status.

Step 507: The control module controls, based on the brain instruction, a first feedback module to implement the action, or controls a second feedback module to intervene in the physiological condition.

In some embodiments, the control module determines, based on the brain instruction, the first feedback module that can implement the action, and sends an action instruction to the first feedback module. The first feedback module receives the action instruction, and implements the action based on the action instruction.

For example, the brain instruction is an instruction used to do housework. The control module determines a robot configured to do housework, and sends an action command to the robot. The robot receives the action command, and performs, based on the action command, an operation of doing housework.

For another example, the brain instruction is an instruction used to watch TV. The control module determines a television for watching TV, and sends a start command to the television. The television receives the start command. The television starts based on the start command, and plays a video.

In some embodiments, the control module determines, based on the first brain state, the second feedback module that can intervene in the physiological condition, and sends a control command to the second feedback module. The second feedback module receives the control command, and intervenes in, based on the control command, the physiological condition that occurs in the brain. Optionally, the second feedback module includes a first intervention parameter, and the first intervention parameter includes one or more of the following information: a width of an intervention signal, an amplitude of the intervention signal, or the like. When triggered by the control command, the second feedback module generates the intervention signal based on the first intervention parameter, and transmits the intervention signal to the brain. The intervention signal may be used to intervene in the physiological condition that occurs in the brain.

For example, the physiological condition that occurs in the brain is epilepsy, the first brain state is an epilepsy state, and the first intervention parameter in the control module includes a width and/or an amplitude of a pulse signal used to treat the epilepsy, and the like. The control module determines, based on the epilepsy state, a nerve stimulator that can treat the epilepsy, and sends the control command to the nerve stimulator. The nerve stimulator receives the control command, generates the pulse signal based on the first intervention parameter, and sends the pulse signal to the brain.

For another example, the physiological condition that occurs in the brain is insomnia, the first brain state is an insomnia state, and the first intervention parameter in the control module includes a width and/or an amplitude of a brain wave signal used to assist in sleep, and the like. The control module determines, based on the insomnia state, a sleep aid device that can assist in sleep, and sends the control command to the sleep aid device. The sleep aid device receives the control command, generates the brain wave signal based on the first intervention parameter, and sends the brain wave signal to the brain.

In some embodiments, the first intervention parameter may be improper, and the first intervention parameter needs to be adjusted. A proper intervention parameter may be obtained through adjustment by using the following procedure including 1 to 4.
1. The signal collection module collects a second neural signal of the brain, converts the second neural signal into a second signal, and sends the second signal to the control module. The second neural signal is collected after the first neural signal is collected.

For a detailed implementation process in which the signal collection module obtains the second signal, refer to related content in step 501. Details are not described herein again.

2. The control module receives the second signal, and obtains a second brain state based on the second signal.

The control module determines a status of the second signal, where the second signal is in the normal state, increases a signal-to-noise ratio of the second signal, and processes, by using the decoding algorithm, the second signal with an increased signal-to-noise ratio, to obtain the second brain state.
3. The control device determines, based on the second brain state, whether the physiological condition still occurs in the brain. If the physiological condition still occurs in the brain, the control device performs the following operation 4. If the physiological condition does not occur in the brain, the control device returns to perform the operation 1.
4. The control device adjusts the first intervention parameter in the second feedback module to a second intervention parameter.

In some embodiments, the control device obtains the first intervention parameter from the second feedback module, decreases the first intervention parameter to obtain the second intervention parameter or increases the first intervention parameter to obtain the second intervention parameter, and updates the first intervention parameter in the second feedback module to the second intervention parameter.

In this way, when an intervention parameter in the second feedback module is improper, the intervention parameter in the second feedback module may be automatically adjusted by using the foregoing operations 1 to 4. In comparison with manual adjustment, this improves adjustment efficiency, and reduces difficulty in adjusting the intervention parameter.

In this embodiment of this application, the signal collection module includes the first configuration parameter. The signal collection module collects the first neural signal of the brain, converts the first neural signal into the first signal based on the first configuration parameter, and sends the first signal to the control module. The control module receives the first signal, obtains the status of the first signal, and when the first signal is in the abnormal state, sends the first information to the signal collection module. The signal collection module receives the first information, and adjusts the first configuration parameter to the second configuration parameter based on the first information, to obtain the proper configuration parameter. In this way, the signal collection module can obtain, based on the second configuration parameter, the first signal in the normal state, and send the first signal in the normal state to the control module, so that the control module can obtain the brain instruction or the brain status based on the first signal in the normal state, to successfully control the feedback module.

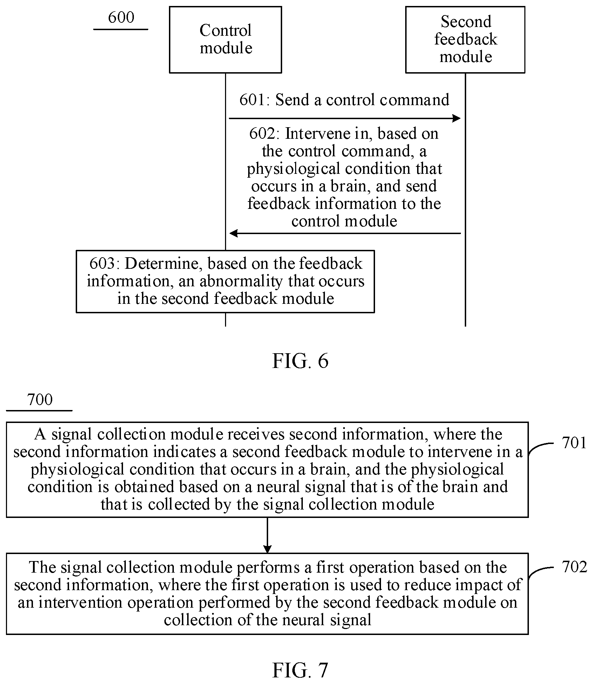

Refer to FIG. 6. An embodiment of this application provides an information processing method 600. The method 600 is applied to the brain-computer system 100 shown in FIG. 1, FIG. 2, FIG. 3, or FIG. 4. When a second feedback module is abnormal, the method 600 may be used to determine an abnormality that occurs in the second feedback module. The method 600 includes the following procedure.

Step 601: A control module sends a control command to the second feedback module, where the control command indicates the second feedback module to intervene in a physiological condition that occurs in a brain.

The control module obtains a first brain state by using steps 501 to 506 in the method 500 shown in FIG. 5. The first brain state is used to reflect a physiological condition that occurs in the brain. The control module determines, based on the first brain state, the second feedback module that can intervene in the physiological condition, and sends the control command to the second feedback module.

For example, the physiological condition that occurs in the brain is epilepsy, and the first brain state is an epilepsy state. The control module determines, based on the epilepsy state, a nerve stimulator that can treat the epilepsy, and sends the control command to the nerve stimulator. For another example, the physiological condition that occurs in the brain is insomnia, and the first brain state is an insomnia state. The control module determines, based on the insomnia state, a sleep aid device that can assist in sleep, and sends the control command to the sleep aid device. Step 602: The second feedback module receives the control command, intervenes in, based on the control command, the physiological condition that occurs in the brain, and sends feedback information to the control module, where the feedback information indicates a situation in which the second feedback module intervenes in the physiological condition.

In step 602, the second feedback module includes a first intervention parameter. When triggered by the control command, the second feedback module generates an intervention signal based on the first intervention parameter, and transmits the intervention signal to the brain. The intervention signal may be used to intervene in the physiological condition that occurs in the brain.

For example, the nerve stimulator receives the control command, generates a pulse signal based on the first intervention parameter, and sends the pulse signal to the brain.

For another example, the sleep aid device receives the control command, generates a brain wave signal based on the first intervention parameter, and sends the brain wave signal to the brain. The second feedback module may be abnormal. The abnormality means that the second feedback device does not intervene in the physiological condition, or the abnormality means that the second feedback device incorrectly intervenes in the physiological condition. To determine the abnormality that occurs in the second feedback module, the second feedback module sends the feedback information to the control device.

In some embodiments, the feedback information includes first indication information, second indication information, or the first intervention parameter. The first indication information indicates that the second feedback device does not intervene in the physiological condition, the second indication information indicates whether the first intervention parameter is the same as a second intervention parameter, the first intervention parameter is a parameter expected to be used by the second feedback module to intervene in the physiological condition, and the second intervention parameter is a parameter actually used by the second feedback module to intervene in the physiological condition.

In some embodiments, refer to FIG. 4. The second feedback module includes a control driving unit and a monitoring unit, the control driving unit includes the first intervention parameter, and the monitoring unit monitors an output of the control driving unit.

The control driving unit receives the control command, and when triggered by the control command, generates the intervention signal, and transmits the intervention signal to the brain. The intervention signal may be used to intervene in the physiological condition that occurs in the brain. Alternatively, after receiving the control command, the control driving unit does not generate the intervention signal.

In some embodiments, when detecting that the control driving unit transmits the intervention signal to the brain, the monitoring unit obtains an intervention parameter of the intervention signal. For ease of description, the intervention parameter of the intervention signal is referred to as the second intervention parameter, and the monitoring unit sends the feedback information to the control module. The feedback information includes the second intervention parameter. The first intervention parameter is the expected intervention parameter. Alternatively, the monitoring unit compares the first intervention parameter with the second intervention parameter, and sends the feedback information to the control module. The feedback information includes the second indication information, and the second indication information indicates whether the first intervention parameter is the same as the second intervention parameter.

The second intervention parameter may be different from the first intervention parameter, and it indicates that the intervention signal generated by the control driving unit is different from an expected intervention signal, that is, the second feedback module incorrectly intervenes in the physiological condition.

In some embodiments, when the monitoring unit detects that the control driving unit does not transmit the intervention signal to the brain, it indicates that the second feedback module does not generate the intervention signal, that is, the second feedback module does not intervene in the physiological condition. The feedback information is sent to the control module, where the feedback information includes the first indication information, and the first indication information indicates that the second feedback module does not intervene in the physiological condition.

Step 603: The control module receives the feedback information, and determines, based on the feedback information, the abnormality that occurs in the second feedback module.

In some embodiments, the feedback information includes the first indication information, and the control module determines, based on the first indication information, that the abnormality that occurs in the second feedback module is that the second feedback module does not intervene in the physiological condition.

In some embodiments, the feedback information includes the second indication information, and the control module determines, based on the second indication information, that the abnormality that occurs in the second feedback module is that the second feedback module incorrectly intervenes in the physiological condition.

In some embodiments, the feedback information includes the second intervention parameter. The control module compares the first intervention parameter with the second intervention parameter to obtain a comparison result. When the comparison result is that the first intervention parameter is different from the second intervention parameter, it is determined that the abnormality that occurs in the second feedback module is that the second feedback module incorrectly intervenes in the physiological condition.

In some embodiments, the control module may display, to a user, the abnormality that occurs in the second feedback module.

In some embodiments, when intervening in the physiological condition, the second feedback module may further send second information to a signal collection module, where the second information indicates the second feedback module to intervene in the physiological condition.

In this embodiment of this application, the signal collection module collects a first neural signal of the brain, converts the first neural signal into a first signal, and sends the first signal to the control module. The control module receives the first signal, obtains a status of the first signal, and when the first signal is in a normal state, obtains the first brain state based on the first signal, and sends the control command to the second feedback module based on the first brain state. The second feedback module receives the control command, and may intervene in, based on the control command, the physiological condition that occurs in the brain, or may not intervene in the physiological condition that occurs in the brain. The second feedback module sends the feedback information to the control module, where the feedback information indicates the situation in which the second feedback module intervenes in the physiological condition. The control module determines, based on the feedback information, the abnormality that occurs in the second feedback module, so that the abnormality can be positioned.

Refer to FIG. 7. An embodiment of this application provides an information processing method 700. The method 700 is applied to the brain-computer system 100 shown in FIG. 1, FIG. 2, FIG. 3, or FIG. 4. When a second feedback module intervenes in a physiological condition that occurs in a brain, a signal collection module may perform a first operation by using the method 700. The method 700 includes the following procedure.

Step 701: The signal collection module receives second information, where the second information indicates the second feedback module to intervene in the physiological condition that occurs in the brain, and the physiological condition is obtained based on a neural signal that is of the brain and that is collected by the signal collection module.

The control module obtains a first brain state by using steps 501 to 506 in the method 500 shown in FIG. 5. The first brain state is used to reflect the physiological condition that occurs in the brain, and sends a control command to the second feedback module. The second feedback module receives the control command, intervenes in, based on the control command, the physiological condition that occurs in the brain, and sends the second information to the signal collection module. In some embodiments, refer to FIG. 4. The second feedback module includes a control driving unit and a monitoring unit, the control driving unit includes a first intervention parameter, and the monitoring unit monitors an output of the control driving unit.

The control driving unit receives the control command, and when triggered by the control command, generates an intervention signal, and transmits the intervention signal to the brain. The intervention signal may be used to intervene in the physiological condition that occurs in the brain. When detecting that the control driving unit transmits the intervention signal to the brain, the monitoring unit sends the second information to the signal collection module.

Because the control driving unit transmits the intervention signal to the brain, the intervention signal may affect the neural signal collected by the signal collection module, and the neural signal may include large noise. In this way, a first signal obtained by converting the neural signal includes large noise, which may affect precision of obtaining, by the control module, a brain status based on the first signal, to control the second feedback module to perform incorrect intervention.

Step 702: The signal collection module performs the first operation based on the second information, where the first operation is used to reduce impact of an intervention operation performed by the second feedback module on collection of the neural signal.

In some embodiments, the first operation includes one or more of the following: disabling a function of collecting the neural signal, or decreasing an amplification multiple for the neural signal.

In step 702, the signal collection module stops collecting the neural signal of the brain, to disable the function of collecting the neural signal. Alternatively,
the signal collection module decreases the amplification multiple for the neural signal. In this way, the signal collection module collects the neural signal, and converts the neural signal into the first signal based on a decreased amplification multiple. An amplitude of the first signal may be less than a second amplitude threshold, so that an abnormality may occur in the first signal. In this way, the control module stops controlling the second feedback module to continue to intervene in the physiological condition of the brain, to avoid controlling the second feedback module to perform incorrect intervention.

In this embodiment of this application, the signal collection module collects a first neural signal of the brain, converts the first neural signal into the first signal, and sends the first signal to the control module. The control module receives the first signal, obtains a status of the first signal, and when the first signal is in a normal state, obtains the first brain state based on the first signal, and sends the control command to the second feedback module based on the first brain state. The second feedback module receives the control command, and may intervene in, based on the control command, the physiological condition that occurs in the brain. The second feedback module sends the second information to the signal collection module, and performs the first operation based on the second information. The first operation includes one or more of the following: disabling the function of collecting the neural signal, or decreasing the amplification multiple for the neural signal. In this way, the control module can stop controlling the second feedback module to continue to intervene in the physiological condition of the brain, to avoid controlling the second feedback module to perform incorrect intervention.

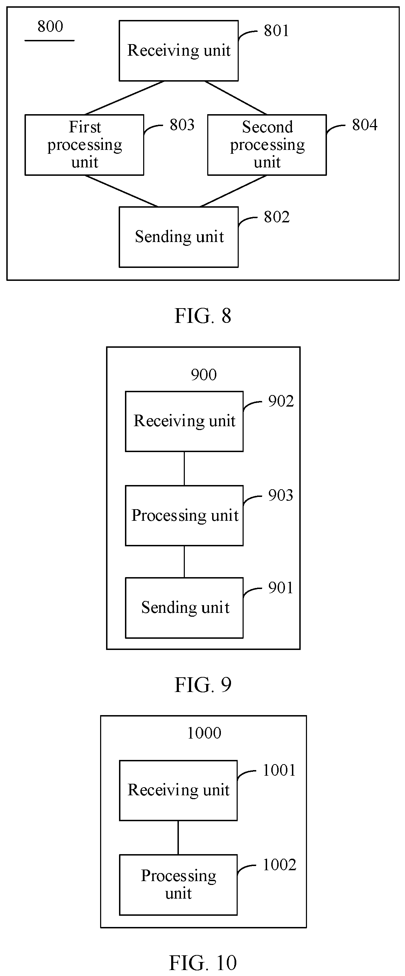

Refer to FIG. 8. An embodiment of this application provides an information processing apparatus 800. The apparatus 800 is used in a brain-computer system, the brain-computer system includes the apparatus 800, and the apparatus 800 includes:
a receiving unit 801, configured to receive a first signal sent by a signal collection module, where the first signal is obtained by the signal collection module by converting a first neural signal of a brain based on a first configuration parameter; and
a sending unit 802, configured to: when the first signal is in an abnormal state, send first information, where the first information indicates the signal collection module to adjust the first configuration parameter to a second configuration parameter.

Optionally, for a detailed implementation process in which the receiving unit 801 receives the first signal, refer to related content of step 503 shown in FIG. 5. Details are not described herein again. Optionally, for a detailed implementation process in which the sending unit 802 sends the first information, refer to related content of step 504 shown in FIG. 5. Details are not described herein again.

Optionally, the abnormal state includes at least one of signal saturation, low signal amplitude, and an oscillation state.

Optionally, the apparatus 800 further includes a threshold determining unit, a mode determining unit, and a storage unit (not shown in the figure).

The threshold determining unit is configured to: compare an amplitude threshold with an amplitude of the first signal to obtain a comparison result, where the amplitude threshold includes a first amplitude threshold and a second amplitude threshold, and the first amplitude threshold is greater than the second amplitude threshold; store the comparison result in the storage unit; and when the comparison result is that the amplitude of the first signal exceeds the first amplitude threshold, determine a status of the first signal, where the status of the first signal includes signal saturation, the storage unit stores a plurality of comparison results, and the plurality of comparison results are comparison results obtained by the threshold determining unit at different time.

The mode determining unit is configured to: when determining, based on the storage unit, that the signal saturation periodically occurs, determine that the status of the first signal further includes an oscillation state; or when determining, based on the storage unit, that a plurality of consecutive comparison results are all that the amplitude of the first signal is less than the second amplitude threshold, determine that the status of the first signal includes low signal amplitude.

Optionally, the signal collection module includes a main control unit, a calculation unit, and a signal conversion unit, and the first signal is obtained by the signal conversion unit by converting the first neural signal based on the first configuration parameter.

The first information indicates the main control unit to send an adjustment manner to the calculation unit based on the status.

The adjustment manner indicates the calculation unit to adjust the first configuration parameter to the second configuration parameter.

Optionally, the apparatus 800 further includes:
a first processing unit 803, configured to: when the first signal is in a normal state, obtain a brain instruction based on the first signal, where the brain instruction indicates an action that needs to be performed for the brain.

The first processing unit 803 is further configured to control, based on the brain instruction, a feedback module to implement the action.

Optionally, for a detailed implementation process in which the first processing unit 803 obtains the brain instruction based on the first signal, refer to related content of step 506 shown in FIG. 5. Details are not described herein again.

Optionally, for a detailed implementation process in which the first processing unit 803 controls the feedback module based on the brain instruction to implement the action, refer to related content of step 507 shown in FIG. 5. Details are not described herein again.

Optionally, the apparatus 800 further includes:
a second processing unit 804, configured to: when the first signal is in a normal state, obtain a first brain state based on the first signal, where the first brain state is used to reflect a physiological condition that occurs in the brain.

The second processing unit 804 is further configured to control, based on the first brain state, the feedback module to intervene in the physiological condition.

Optionally, for a detailed implementation process in which the second processing unit 804 obtains the first brain state based on the first signal, refer to related content of step 506 shown in FIG. 5. Details are not described herein again.

Optionally, for a detailed implementation process in which the second processing unit 804 controls, based on the first brain state, the feedback module to intervene in the physiological condition, refer to related content of step 507 shown in FIG. 5. Details are not described herein again.

Optionally, the receiving unit 801 is further configured to receive a second signal, where the second signal is obtained by the signal collection module by converting a second neural signal generated by the brain, and the second neural signal is collected after the first neural signal is collected.

The second processing unit 804 is further configured to obtain a second brain state based on the second signal.

The second processing unit 804 is further configured to: when determining, based on the second brain state, that the physiological condition still occurs in the brain, adjust an intervention parameter in the feedback module, where the intervention parameter is a parameter used by the feedback module to intervene in the physiological condition.

Optionally, for a detailed implementation process in which the receiving unit 801 receives the second signal, refer to related content of step 507 shown in FIG. 5. Details are not described herein again.

Optionally, for a detailed implementation process in which the second processing unit 804 obtains the second brain state based on the second signal, refer to related content of step 507 shown in FIG. 5. Details are not described herein again.

Optionally, for a detailed implementation process in which the second processing unit 804 adjusts the intervention parameter in the feedback module, refer to related content of step 507 shown in FIG. 5. Details are not described herein again.

Optionally, the apparatus 800 and the signal collection module are integrated into one device.

In this embodiment of this application, the control module receives the first signal sent by the signal collection module, where the first signal is obtained by the signal collection module by converting the first neural signal of the brain based on the first configuration parameter. When the first signal is in the abnormal state, the control module sends the first information, where the first information indicates the signal collection module to adjust the first configuration parameter to the second configuration parameter. Because the control module sends the first information when the first signal is in the abnormal state, where the first information indicates the signal collection module to adjust the first configuration parameter to the second configuration parameter, a proper configuration parameter can be obtained through adjustment. In this way, the signal collection module obtains the first signal in a normal state through conversion based on the proper configuration parameter, to ensure that the brain instruction or a brain status can be obtained based on the first signal, and ensure that the feedback module can be controlled.

Refer to FIG. 9. An embodiment of this application provides an information processing apparatus 900. The apparatus 900 is used in a brain-computer system, the brain-computer system includes the apparatus 900, and the apparatus 900 includes:
a sending unit 901, configured to send a control command, where the control command indicates a feedback module to intervene in a physiological condition that occurs in a brain;
a receiving unit 902, configured to receive feedback information, where the feedback information indicates a situation in which the feedback module intervenes in the physiological condition; and
a processing unit 903, configured to determine, based on the feedback information, an abnormality that occurs in the feedback module.

Optionally, for a detailed implementation process in which the sending unit 901 sends the control command, refer to related content of step 601 shown in FIG. 6. Details are not described herein again.

Optionally, for a detailed implementation process in which the receiving unit 902 receives the feedback information, refer to related content of step 603 shown in FIG. 6. Details are not described herein again.

Optionally, for a detailed implementation process in which the processing unit 903 determines, based on the feedback information, the abnormality that occurs in the feedback module, refer to related content of step 603 shown in FIG. 6. Details are not described herein again.

Optionally, the abnormality means that the feedback module does not intervene in the physiological condition, or the abnormality means that the feedback module incorrectly intervenes in the physiological condition.

Optionally, the abnormality means that the feedback module incorrectly intervenes in the physiological condition, the feedback information indicates whether a first intervention parameter is the same as a second intervention parameter, the first intervention parameter is a parameter expected to be used by the feedback module to intervene in the physiological condition, and the second intervention parameter is a parameter actually used by the feedback module to intervene in the physiological condition.

The processing unit 903 is configured to: when determining, based on the feedback information, that the first intervention parameter is different from the second intervention parameter, determine that the feedback module incorrectly intervenes in the physiological condition.

Optionally, the feedback information includes the second intervention parameter; or the feedback module includes the first intervention parameter, and the feedback information includes a comparison result of comparing the first intervention parameter with the second intervention parameter by the feedback module.

Optionally, the feedback module includes a control driving unit and a monitoring unit. The control driving unit is configured to intervene in the physiological condition. The monitoring unit is configured to: when detecting that the control driving unit intervenes in the physiological condition, obtain the second intervention parameter, and send the feedback information, where the feedback information includes the second intervention parameter, or a comparison result of comparing the first intervention parameter with the second intervention parameter.

In this embodiment of this application, the control module sends the control command, where the control command indicates the feedback module to intervene in the physiological condition that occurs in the brain. The control module receives the feedback information, where the feedback information indicates the situation in which the feedback module intervenes in the physiological condition. The control module determines, based on the feedback information, the abnormality that occurs in the feedback module. The feedback module sends the feedback information to the control module, where the feedback information indicates the situation in which the feedback module intervenes in the physiological condition. The control module determines, based on the feedback information, the abnormality that occurs in the feedback module, so that the abnormality can be positioned.

Refer to FIG. 10. An embodiment of this application provides an information processing apparatus 1000. The apparatus 1000 is used in a brain-computer system, the brain-computer system includes the apparatus 1000, and the apparatus 1000 includes:
a receiving unit 1001, configured to receive second information, where the second information indicates a feedback module to intervene in a physiological condition that occurs in a brain, and the physiological condition is obtained based on a neural signal that is of the brain and that is collected by the apparatus 1000; and
a processing unit 1002, configured to perform a first operation based on the second information, where the first operation is used to reduce impact of an intervention operation performed by the feedback module on collection of the neural signal.

Optionally, for a detailed implementation process in which the receiving unit 1001 receives the second information, refer to related content of step 701 shown in FIG. 7. Details are not described herein again.

Optionally, for a detailed implementation process in which the processing unit 1002 performs the first operation based on the second information, refer to related content of step 702 shown in FIG. 7. Details are not described herein again.

Optionally, the first operation includes one or more of the following: disabling a function of collecting the neural signal, or decreasing an amplification multiple for the neural signal.

In this embodiment of this application, the feedback module sends the second information to the signal collection module, and performs the first operation based on the second information. The first operation includes one or more of the following: disabling the function of collecting the neural signal, or decreasing the amplification multiple for the neural signal. In this way, a control module can stop controlling the feedback module to continue to intervene in the physiological condition of the brain, to avoid controlling the feedback module to perform incorrect intervention.

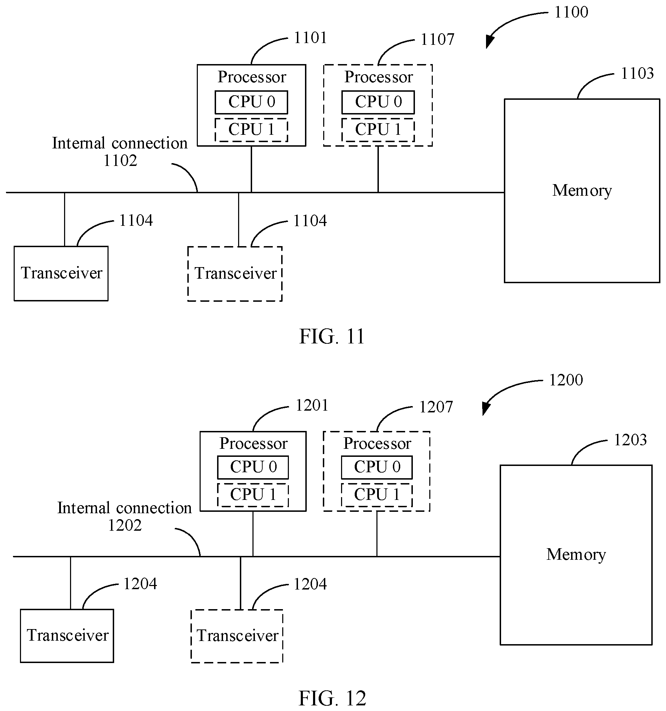

FIG. 11 is a diagram of an information processing apparatus 1100 according to an embodiment of this application. The apparatus 1100 may be the control module in any one of the foregoing embodiments. The apparatus 1100 includes at least one processor 1101, an internal connection 1102, a memory 1103, and at least one transceiver 1104.

The apparatus 1100 is an apparatus of a hardware structure, and may be configured to implement functional modules in the apparatus 800 shown in FIG. 8. For example, a person skilled in the art may figure out that the first processing unit 803 and the second processing unit 804 in the apparatus 800 shown in FIG. 8 may be implemented by the at least one processor 1101 by invoking code in the memory 1103, and the receiving unit 801 and the sending unit 802 in the apparatus 800 shown in FIG. 8 may be implemented by the transceiver 1104. Alternatively,
the apparatus 1100 is an apparatus of a hardware structure, and may be configured to implement functional modules in the apparatus 900 in FIG. 9. For example, a person skilled in the art may figure out that the processing unit 903 in the apparatus 900 shown in FIG. 9 may be implemented by the at least one processor 1101 by invoking code in the memory 1103, and the sending unit 901 and the receiving unit 902 in the apparatus 900 shown in FIG. 9 may be implemented by the transceiver 1104.

Optionally, the apparatus 1100 may be further configured to implement a function of the control module in any one of the foregoing embodiments.

Optionally, the processor 1101 may be a general-purpose central processing unit (central processing unit, CPU), a network processor (network processor, NP), a microprocessor, an application-specific integrated circuit (application-specific integrated circuit, ASIC), or one or more integrated circuits configured to control program execution of the solutions in this application.

The internal connection 1102 may include a path for transmitting information between the foregoing components. Optionally, the internal connection 1102 is a board, a bus, or the like. The transceiver 1104 is configured to communicate with another device or a communication network.

The memory 1103 may be a read-only memory (read-only memory, ROM) or another type of static storage device that can store static information and instructions, or a random access memory (random access memory, RAM) or another type of dynamic storage device that can store information and instructions; or may be an electrically erasable programmable read-only memory (electrically erasable programmable read-only memory, EEPROM), a compact disc read-only memory (compact disc read-only memory, CD-ROM) or another optical disc storage, an optical disc storage (including a compact optical disc, a laser disc, an optical disc, a digital versatile disc, a Blu-ray disc, or the like), a magnetic disk storage medium or another magnetic storage device, or any other medium that can be configured to carry or store expected program code in a form of instructions or a data structure and that can be accessed by a computer. This is not limited thereto. The memory may exist independently, and is connected to the processor through the bus. The memory may alternatively be integrated with the processor.

The memory 1103 is configured to store application program code for performing the solutions in this application, and the processor 1101 controls execution of the application program code. The processor 1101 is configured to execute the application program code stored in the memory 1103, and cooperate with the at least one transceiver 1104, so that the apparatus 1100 implements functions in the method in this patent.

During specific implementation, in an embodiment, the processor 1101 may include one or more CPUs such as a CPU 0 and a CPU 1 in FIG. 11.

During specific implementation, in an embodiment, the apparatus 1100 may include a plurality of processors, such as the processor 1101 and a processor 1107 in FIG. 11. Each of the processors may be a single-core (single-CPU) processor, or may be a multi-core (multi-CPU) processor. The processor herein may be one or more devices, circuits, and/or processing cores configured to process data (for example, computer program instructions).

FIG. 12 is a diagram of an information processing apparatus 1200 according to an embodiment of this application. The apparatus 1200 may be the signal collection module in any one of the foregoing embodiments. The apparatus 1200 includes at least one processor 1201, an internal connection 1202, a memory 1203, and at least one transceiver 1204.

The apparatus 1200 is an apparatus of a hardware structure, and may be configured to implement functional modules in the apparatus 1000 in FIG. 10. For example, a person skilled in the art may figure out that the processing unit 1002 in the apparatus 1000 shown in FIG. 10 may be implemented by the at least one processor 1201 by invoking code in the memory 1203, and the receiving unit 1001 in the apparatus 1000 shown in FIG. 10 may be implemented by the transceiver 1204.

Optionally, the apparatus 1200 may be further configured to implement a function of the signal collection module in any one of the foregoing embodiments.

Optionally, the processor 1201 may be a general-purpose central processing unit (central processing unit, CPU), a network processor (network processor, NP), a microprocessor, an application-specific integrated circuit (application-specific integrated circuit, ASIC), or one or more integrated circuits configured to control program execution of the solutions in this application.

The internal connection 1202 may include a path for transmitting information between the foregoing components. Optionally, the internal connection 1202 is a board, a bus, or the like.

The transceiver 1204 is configured to communicate with another device or a communication network.

The memory 1203 may be a read-only memory (read-only memory, ROM) or another type of static storage device that can store static information and instructions, or a random access memory (random access memory, RAM) or another type of dynamic storage device that can store information and instructions; or may be an electrically erasable programmable read-only memory (electrically erasable programmable read-only memory, EEPROM), a compact disc read-only memory (compact disc read-only memory, CD-ROM) or another optical disc storage, an optical disc storage (including a compact optical disc, a laser disc, an optical disc, a digital versatile disc, a Blu-ray disc, or the like), a magnetic disk storage medium or another magnetic storage device, or any other medium that can be configured to carry or store expected program code in a form of instructions or a data structure and that can be accessed by a computer. This is not limited thereto. The memory may exist independently, and is connected to the processor through the bus. The memory may alternatively be integrated with the processor.

The memory 1203 is configured to store application program code for performing the solutions in this application, and the processor 1201 controls execution of the application program code. The processor 1201 is configured to execute the application program code stored in the memory 1203, and cooperate with the at least one transceiver 1204, so that the apparatus 1200 implements functions in the method in this patent.

During specific implementation, in an embodiment, the processor 1201 may include one or more CPUs such as a CPU 0 and a CPU 1 in FIG. 12.

During specific implementation, in an embodiment, the apparatus 1200 may include a plurality of processors, such as the processor 1201 and a processor 1207 in FIG. 12. Each of the processors may be a single-core (single-CPU) processor, or may be a multi-core (multi-CPU) processor. The processor herein may be one or more devices, circuits, and/or processing cores configured to process data (for example, computer program instructions).

A person of ordinary skill in the art may understand that all or a part of the steps of the foregoing embodiments may be implemented by hardware or a program instructing related hardware. The program may be stored in a computer-readable storage medium. The storage medium mentioned above may be a read-only memory, a magnetic disk, an optical disc, or the like.

The foregoing descriptions are merely optional embodiments of this application, but are not intended to limit this application. Any modification, equivalent replacement, improvement, or the like made within the principle of this application should fall within the protection scope of this application.

## Claims

1. An information processing method, wherein the method is applied to a brain-computer system, the brain-computer system comprises a control module, and the method comprises:
receiving, by the control module, a first signal sent by a signal collection module, wherein the first signal is obtained by the signal collection module by converting a first neural signal of a brain based on a first configuration parameter; and
when the first signal is in an abnormal state, sending, by the control module, first information, wherein the first information indicates the signal collection module to adjust the first configuration parameter to a second configuration parameter.

2. The method according to claim 1, wherein the abnormal state comprises at least one of signal saturation, low signal amplitude, and an oscillation state.

3. The method according to claim 1, wherein the control module comprises a threshold determining unit, a mode determining unit, and a storage unit;
the threshold determining unit is configured to: compare an amplitude threshold with an amplitude of the first signal to obtain a comparison result, wherein the amplitude threshold comprises a first amplitude threshold and a second amplitude threshold, and the first amplitude threshold is greater than the second amplitude threshold; store the comparison result in the storage unit; and when the comparison result is that the amplitude of the first signal exceeds the first amplitude threshold, determine a status of the first signal, wherein the status of the first signal comprises signal saturation, the storage unit stores a plurality of comparison results, and the plurality of comparison results are comparison results obtained by the threshold determining unit at different time; and
the mode determining unit is configured to: when determining, based on the storage unit, that the signal saturation periodically occurs, determine that the status of the first signal further comprises an oscillation state; or when determining, based on the storage unit, that a plurality of consecutive comparison results are all that the amplitude of the first signal is less than the second amplitude threshold, determine that the status of the first signal comprises low signal amplitude.

4. The method according to any one of claims 1 to 3, wherein the signal collection module comprises a main control unit, a calculation unit, and a signal conversion unit, and the first signal is obtained by the signal conversion unit by converting the first neural signal based on the first configuration parameter;
the first information indicates the main control unit to send an adjustment manner to the calculation unit based on the status; and
the adjustment manner indicates the calculation unit to adjust the first configuration parameter to the second configuration parameter.

5. The method according to any one of claims 1 to 4, wherein the method further comprises:
when the first signal is in a normal state, obtaining, by the control module, a brain instruction based on the first signal, wherein the brain instruction indicates an action that needs to be performed for the brain; and
controlling, by the control module based on the brain instruction, a feedback module to implement the action.

6. The method according to any one of claims 1 to 4, wherein the method further comprises:
when the first signal is in a normal state, obtaining, by the control module, a first brain state based on the first signal, wherein the first brain state is used to reflect a physiological condition that occurs in the brain; and
controlling, by the control module based on the first brain state, the feedback module to intervene in the physiological condition.

7. The method according to claim 6, wherein the method further comprises:
receiving, by the control module, a second signal, wherein the second signal is obtained by the signal collection module by converting a second neural signal generated by the brain, and the second neural signal is collected after the first neural signal is collected;
obtaining, by the control module, a second brain state based on the second signal; and
when determining, based on the second brain state, that the physiological condition still occurs in the brain, adjusting, by the control module, an intervention parameter in the feedback module, wherein the intervention parameter is a parameter used by the feedback module to intervene in the physiological condition.

8. The method according to any one of claims 1 to 7, wherein the control module and the signal collection module are integrated into one device.

9. An information processing method, wherein the method is applied to a brain-computer system, the brain-computer system comprises a control module, and the method comprises:
sending, by the control module, a control command, wherein the control command indicates a feedback module to intervene in a physiological condition that occurs in a brain;
receiving, by the control module, feedback information, wherein the feedback information indicates a situation in which the feedback module intervenes in the physiological condition; and
determining, by the control module based on the feedback information, an abnormality that occurs in the feedback module.

10. The method according to claim 9, wherein the abnormality means that the feedback module does not intervene in the physiological condition, or the abnormality means that the feedback module incorrectly intervenes in the physiological condition.

11. The method according to claim 10, wherein the abnormality means that the feedback module incorrectly intervenes in the physiological condition, the feedback information indicates whether a first intervention parameter is the same as a second intervention parameter, the first intervention parameter is a parameter expected to be used by the feedback module to intervene in the physiological condition, and the second intervention parameter is a parameter actually used by the feedback module to intervene in the physiological condition; and
the determining, by the control module based on the feedback information, the abnormality that occurs in the feedback module comprises:
when determining, based on the feedback information, that the first intervention parameter is different from the second intervention parameter, determining, by the control module, that the feedback module incorrectly intervenes in the physiological condition.

12. The method according to claim 11, wherein the feedback information comprises the second intervention parameter; or the feedback module comprises the first intervention parameter, and the feedback information comprises a comparison result of comparing the first intervention parameter with the second intervention parameter by the feedback module.

13. The method according to claim 11, wherein the feedback module comprises a control driving unit and a monitoring unit;
the control driving unit is configured to intervene in the physiological condition; and
the monitoring unit is configured to: when detecting that the control driving unit intervenes in the physiological condition, obtain the second intervention parameter, and send the feedback information, wherein the feedback information comprises the second intervention parameter, or a comparison result of comparing the first intervention parameter with the second intervention parameter.

14. An information processing method, wherein the method is applied to a brain-computer system, the brain-computer system comprises a signal collection module, and the method comprises:
receiving, by the signal collection module, second information, wherein the second information indicates a feedback module to intervene in a physiological condition that occurs in a brain, and the physiological condition is obtained based on a neural signal that is of the brain and that is collected by the signal collection module; and
performing, by the signal collection module, a first operation based on the second information, wherein the first operation is used to reduce impact of an intervention operation performed by the feedback module on collection of the neural signal.

15. The method according to claim 14, wherein the first operation comprises one or more of the following: disabling a function of collecting the neural signal, or decreasing an amplification multiple for the neural signal.

16. An information processing apparatus, wherein the apparatus is used in a brain-computer system, the brain-computer system comprises the apparatus, and the apparatus comprises:
a receiving unit, configured to receive a first signal sent by a signal collection module, wherein the first signal is obtained by the signal collection module by converting a first neural signal of a brain based on a first configuration parameter; and
a sending unit, configured to: when the first signal is in an abnormal state, send first information, wherein the first information indicates the signal collection module to adjust the first configuration parameter to a second configuration parameter.

17. The apparatus according to claim 16, wherein the abnormal state comprises at least one of signal saturation, low signal amplitude, and an oscillation state.

18. The apparatus according to claim 16, wherein the apparatus further comprises a threshold determining unit, a mode determining unit, and a storage unit;
the threshold determining unit is configured to: compare an amplitude threshold with an amplitude of the first signal to obtain a comparison result, wherein the amplitude threshold comprises a first amplitude threshold and a second amplitude threshold, and the first amplitude threshold is greater than the second amplitude threshold; store the comparison result in the storage unit; and when the comparison result is that the amplitude of the first signal exceeds the first amplitude threshold, determine a status of the first signal, wherein the status of the first signal comprises signal saturation, the storage unit stores a plurality of comparison results, and the plurality of comparison results are comparison results obtained by the threshold determining unit at different time; and
the mode determining unit is configured to: when determining, based on the storage unit, that the signal saturation periodically occurs, determine that the status of the first signal further comprises an oscillation state; or when determining, based on the storage unit, that a plurality of consecutive comparison results are all that the amplitude of the first signal is less than the second amplitude threshold, determine that the status of the first signal comprises low signal amplitude.

19. The apparatus according to any one of claims 16 to 18, wherein the signal collection module comprises a main control unit, a calculation unit, and a signal conversion unit, and the first signal is obtained by the signal conversion unit by converting the first neural signal based on the first configuration parameter;
the first information indicates the main control unit to send an adjustment manner to the calculation unit based on the status; and
the adjustment manner indicates the calculation unit to adjust the first configuration parameter to the second configuration parameter.

20. The apparatus according to any one of claims 16 to 19, wherein the apparatus further comprises:
a first processing unit, configured to: when the first signal is in a normal state, obtain a brain instruction based on the first signal, wherein the brain instruction indicates an action that needs to be performed for the brain, wherein
the first processing unit is further configured to control, based on the brain instruction, a feedback module to implement the action.

21. The apparatus according to any one of claims 16 to 19, wherein the apparatus further comprises:
a second processing unit, configured to: when the first signal is in a normal state, obtain a first brain state based on the first signal, wherein the first brain state is used to reflect a physiological condition that occurs in the brain, wherein
the second processing unit is further configured to control, based on the first brain state, the feedback module to intervene in the physiological condition.

22. The apparatus according to claim 21, wherein
the receiving unit is further configured to receive a second signal, wherein the second signal is obtained by the signal collection module by converting a second neural signal generated by the brain, and the second neural signal is collected after the first neural signal is collected;
the second processing unit is further configured to obtain a second brain state based on the second signal; and
the second processing unit is further configured to: when determining, based on the second brain state, that the physiological condition still occurs in the brain, adjust an intervention parameter in the feedback module, wherein the intervention parameter is a parameter used by the feedback module to intervene in the physiological condition.

23. The apparatus according to any one of claims 16 to 22, wherein the apparatus and the signal collection module are integrated into one device.

24. An information processing apparatus, wherein the apparatus is used in a brain-computer system, the brain-computer system comprises the apparatus, and the apparatus comprises:
a sending unit, configured to send a control command, wherein the control command indicates a feedback module to intervene in a physiological condition that occurs in a brain;
a receiving unit, configured to receive feedback information, wherein the feedback information indicates a situation in which the feedback module intervenes in the physiological condition; and
a processing unit, configured to determine, based on the feedback information, an abnormality that occurs in the feedback module.

25. The apparatus according to claim 24, wherein the abnormality means that the feedback module does not intervene in the physiological condition, or the abnormality means that the feedback module incorrectly intervenes in the physiological condition.

26. The apparatus according to claim 25, wherein the abnormality means that the feedback module incorrectly intervenes in the physiological condition, the feedback information indicates whether a first intervention parameter is the same as a second intervention parameter, the first intervention parameter is a parameter expected to be used by the feedback module to intervene in the physiological condition, and the second intervention parameter is a parameter actually used by the feedback module to intervene in the physiological condition; and
the processing unit is configured to: when determining, based on the feedback information, that the first intervention parameter is different from the second intervention parameter, determine that the feedback module incorrectly intervenes in the physiological condition.

27. The apparatus according to claim 26, wherein the feedback information comprises the second intervention parameter; or the feedback module comprises the first intervention parameter, and the feedback information comprises a comparison result of comparing the first intervention parameter with the second intervention parameter by the feedback module.

28. The apparatus according to claim 26, wherein the feedback module comprises a control driving unit and a monitoring unit;
the control driving unit is configured to intervene in the physiological condition; and
the monitoring unit is configured to: when detecting that the control driving unit intervenes in the physiological condition, obtain the second intervention parameter, and send the feedback information, wherein the feedback information comprises the second intervention parameter, or a comparison result of comparing the first intervention parameter with the second intervention parameter.

29. An information processing apparatus, wherein the apparatus is used in a brain-computer system, the brain-computer system comprises the apparatus, and the apparatus comprises:
a receiving unit, configured to receive second information, wherein the second information indicates a feedback module to intervene in a physiological condition that occurs in a brain, and the physiological condition is obtained based on a neural signal that is of the brain and that is collected by the apparatus; and
a processing unit, configured to perform a first operation based on the second information, wherein the first operation is used to reduce impact of an intervention operation performed by the feedback module on collection of the neural signal.

30. The apparatus according to claim 29, wherein the first operation comprises one or more of the following: disabling a function of collecting the neural signal, or decreasing an amplification multiple for the neural signal.

31. A chip, wherein the chip comprises program code, and when the program code is run on the chip, the chip is caused to perform the method according to any one of claims 1 to 15.

32. An information processing system, wherein the system comprises the apparatus according to any one of claims 16 to 28 and the apparatus according to claim 29 or 30.
